(19) Europäisches Patentamt
European Patent Office
Office européen des brevets

(11) Publication number: 0 123 920 B1

(12) # EUROPEAN PATENT SPECIFICATION

(45) Date of publication of patent specification: 20.11.86

(51) Int. Cl.[4]: **C 07 D 233/64,** C 07 D 307/52, C 07 D 277/48, C 07 D 295/08, A 61 K 31/34, A 61 K 31/425, A 61 K 31/40, A 61 K 31/415, A 61 K 31/445, A 61 K 31/535

(21) Application number: 84103490.3

(22) Date of filing: 29.03.84

(54) **N-cyano-formamidine compounds, process for preparing them and pharmaceutical compositions containing them.**

(30) Priority: 30.03.83 ES 521588

(43) Date of publication of application: 07.11.84 Bulletin 84/45

(45) Publication of the grant of the patent: 20.11.86 Bulletin 86/47

(84) Designated Contracting States: BE CH DE FR GB IT LI NL

(58) References cited:
EP-A-0 002 930
EP-A-0 030 092
AT-B- 363 955
GB-A-1 565 966

(73) Proprietor: **FERRER INTERNACIONAL, S.A.**
**Gran Via Carlos III, 94**
**08028 Barcelona (ES)**

(72) Inventor: **Foguet, Rafael**
**Llusanés 8**
**Barcelona 22 (ES)**
Inventor: **Anglada, Luis**
**Bruch 111**
**Barcelona 9 (ES)**
Inventor: **Raga, Manuel M.**
**Sors 17/20**
**Barcelona 24 (ES)**
Inventor: **Ortiz, José A.**
**Córcega 429**
**Barcelona 37 (ES)**
Inventor: **Sacristán, Aurelio**
**Santa Amelia 2**
**Barcelona 34 (ES)**
Inventor: **Castelló, José M.**
**Av. Principe de Asturias 35**
**Barcelona 12 (ES)**

(74) Representative: **Kinzebach, Werner, Dr.**
**Patentanwälte Reitstötter, Kinzebach und Partner Sternwartstrasse 4 Postfach 86 06 49**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

**Description**

The present invention relates to new N-cyano-formamidine compounds, a process for preparing them and pharmaceutical compositions containing them. The compounds of the invention are histamine $H_2$-receptor antagonists and are therapeutically useful as acid secretion inhibitors.

The compounds of the present invention have the general formula I:

N—CN
||
H—C—NH—R    I

wherein R is a radical of the general formulae:

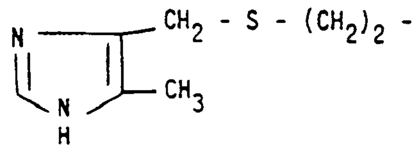

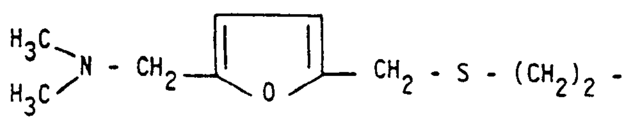

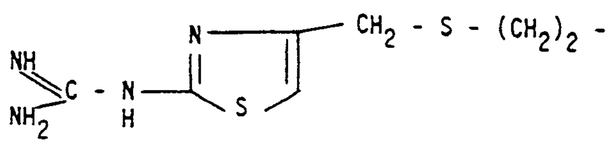

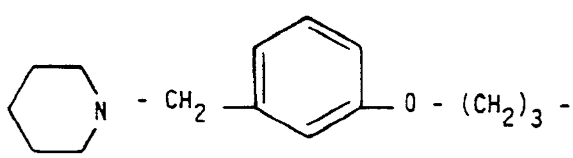

Useful non-toxic, pharmaceutically acceptable addition salts include salts with inorganic or organic acids, such as hydrogen chloride, hydrogen bromide, sulfuric acid, phosphoric acid, maleic acid, malic acid, citronic acid, lactic acid and tartaric acid. The present invention also comprises salts with more than 1 molar equivalent of acid.

The process for preparing the compounds of the invention is characterized in that a N-Cyano-formimidate of the general formula II

N—CN
||
H—C—$OR_1$    II

wherein $R_1$ is a linear or branched $C_1$—$C_4$ alkyl group, is reacted with a compound of the general formula III

$R—NH_2$    III

wherein R is as defined above, in an inert organic reaction medium and the so obtained compound optionally is converted into a non-toxic acid addition salt thereof.

The organic reaction medium is preferably selected from acetonitrile or an $C_1$—$C_4$ alkanol. The mostly preferred medium is ethanol.

It is preferred to conduct the above reaction at about room temperature.

The amines of the formula III also can be used as salts with mineral acids such as hydrochlorides or dihydrochlorides. In this case it is convenient to neutralize the acid before starting the reaction with N-cyano-formimidate of the formula II. This can be done by adding an organic base, preferably triethylamine, or an aqueous solution of an alkaline metal or alkaline earthmetal carbonate or bicarbonate, preferably potassium carbonate.

The preparation of the acid addition salts of the compounds of the present invention may be carried out by reacting the present compounds as free bases with one or more molar equivalents of acid in an inert medium which is preferably selected from ethanol, acetone, ethyl ether or mixtures thereof.

If required the compounds of the present invention finally may be purified for example by crystallization.

The compounds of the present invention are remarkably capable of antagonizing histamine $H_2$-receptors at an acceptable toxicity which makes them therapeutically useful as acid secretion inhibitors.

In effect, it is known that histamine, a physiological compound which is found in living organisms, may be bound to certain specific receptors for exerting its activity. Two classes of histamine receptors have been identified so far: $H_1$-receptors (Ash and Schild: Brit.J.Pharmac., *20*, 427, 1966), in which the histamine activity is blocked to conventional antihistaminic drugs like mepyramine; and $H_2$-receptors (Black et al., Nature, *236*, 385, 1972) in which the histamine activity is blocked by cimetidine. The blockade of histamine activity by $H_2$-receptors results in the inhibition of gastric acid secretion, thus making the compounds with this potency effective for the treatment of peptic ulcers and other pathologies caused or stimulated by gastric acidity.

The blocking activity of histamine $H_2$-receptors was tested in Sprague-Dawley albino male rats weighing 350—450 g. Animals which were fasted for 18 hours were anaesthetized with urethane (1.5 g/kg i.p.), then a longitudinal incision was performed on the abdominal wall and pylorus and esophagus were cannulated for subsequent perfusion. Also the femoral veins were cannulated for administration of test drugs and histamine solution.

The stomach of the animals was perfused with 1/8000 N NaOH diluted solution at a rate of 2 ml/min. The perfusion liquid was circulated through a 3 ml volume chamber provided with a pH meter electrode. After calibrating the pH, histamine was perfused at 0.325 µmoles/kg/min.

Histamine perfusion caused a decrease of the pH of the perfusion liquid which was inhibited by the compounds of the present invention.

The results, expressed as the inhibition rate of maximal pH decrease in relation to initial pH, demonstrate that the compounds of the present invention: N - cyano - N' - [2[[[2 - [(aminomethyl)amino] - 4 - thiazolyl]methyl]thio]ethyl]formamidine maleate acid (Example 3) and N - cyano - N' - [3 - [3 - (1 - piperidinylmethyl)phenoxy]propyl]formamidine hydrochloride (Example 4) are, according to their $ED_{50}$ values (Table 1), 2.6 and 1.8 times, respectively, more active than Cimetidine.

TABLE 1

| Compound | $ED_{50}$ (µmols/kg) |
|---|---|
| From Example 3 | 2.17 |
| From Example 4 | 3.20 |
| Cimetidine | 5.60 |

The compounds of the present invention mixed with pharmaceutically acceptable carriers can be administered by the oral route in the form of tablets, capsules, syrup, solution, etc., by injectable route and by rectal route, at daily doses ranging from 5 to 2000 mg.

A number of examples will now be described in non-limitative manner to illustrate the invention.

Example 1

N-Cyano-N'-[2-[[(5-methyl-1H-imidazol-4-yl)methyl]thio]ethyl]formamidine dihydrochloride

I.

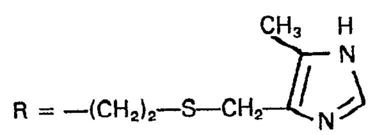

. 2HCl

To a solution of 4.88 g of 2 - [[(5 - methyl - 1H - imidazol - 4- yl) - methyl]thio]ethanamine (prepared

according to British Patent No. 1,338,169) in 75 ml of absolute ethanol, 5.55 ml of triethylamine are added. Then, 1.96 g of ethyl N-cyano-formimidate (prepared according to Huffmann and Schaefer "J.Org.Chem.", *28,* 1816, 1963) in 15 ml of ethanol are added dropwise with stirring at room temperature. Stirring is maintained for 1 hour and then 2.5 ml of 8N HCl/ethanol are added. A white solid is separated which is subsequently crystallized in ethanol, filtered off and dried in a vacuum desiccator in the presence of phosphorus pentoxide and sodium hydroxide. 2.9 g of N - cyano - N' - [2 - [[(5 - methyl - 1H - imidazol - 4 - yl)methyl]thio]ethyl]formamidine dihydrochloride are obtained.

Melting point: 168—170°C.

| Elemememtal analysis: | C | H | N |
|---|---|---|---|
| found | 40.89 | 5.48 | 25.7 |
| calculated | 41.62 | 5.39 | 26.47 |

| | found | calculated |
|---|---|---|
| Sulfur (Schöniger): | 12.34 | 12.34 |
| Chlorides: | 14.6 | 13.68 |

IR Spectrum: characteristic bands at 2190 $cm^{-1}$ (—C≡N st) and 1615 $cm^{-1}$ (—C=N st).

## Example 2

N-Cyano-N'-[2-[[[5-[(dimethylamino)methyl]-2-furanyl]methyl]thio]ethyl]formamidine

I. R = 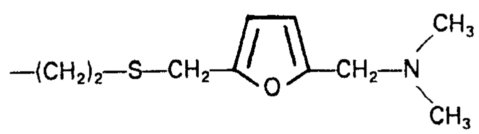

To a solution of 5.6 g of 5 - [[(2 - aminoethyl)thio]methyl] - N,N - dimethylfuranmethanamine (prepared according to Belgian Patent No. 857,388) in 35 ml of acetonitrile and under cooling at 0°C, 2.56 g of N-cyano-formimidate in 15 ml of acetonitrile are added dropwise. The mixture is kept with stirring for 30 minutes and the acetonitrile is removed by vacuum evaporation. A solid residue is formed which is crystallized from ethyl acetate thus yielding 2.1 g of N - cyano - N' - [2 - [[[5 - [(dimethylamino)methyl] - 2-furanyl]methyl]thio]ethyl]formamidine.

Melting point: 74—76°C.

One basic group (anhydrous medium): 100.3%

| Elemental analysis | C | H | N |
|---|---|---|---|
| found | 54.49 | 6.60 | 21.02 |
| calculated | 54.11 | 6.81 | 21.04 |

| | found | calculated |
|---|---|---|
| Sulfur (Schöniger): | 12.27 | 12.04 |

IR Spectrum: characteristic bands at 2200 $cm^{-1}$ (C≡N st) and 1630 $cm^{-1}$ (C=N st).

## Example 3

N-Cyano-N'-[2-[[[2-(aminoiminomethyl)amino]-4-thiazolyl]methyl]thio]ethyl]formamidine acid maleate

I. R = 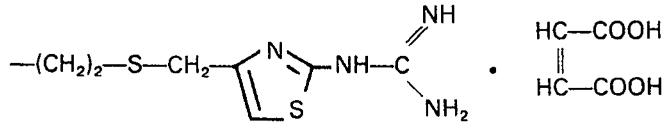

To a solution of 1.82 g of [4 - [[(2 - aminoethyl)thio]methyl] - 2 - thiazolyl]guanidine dihydrochloride (prepared according to US Patent No. 4,165,378) in 20 ml of water, 0.83 g of potassium carbonate are added. The resulting solution is evaporated under vacuum and the residue obtained is treated with 20 ml of absolute ethanol. The insoluble solid is removed and 0.54 g of N-cyano-formimidate in 10 ml of ethanol are

added dropwise to the filtrate with stirring at room temperature. Stirring is continued for 1 hour and the solvent is removed by distillation under reduced pressure and the residue obtained is dissolved in 25 ml of acetone. Then 1.4 g of maleic acid in 15 ml of acetone are added resulting in a white solid which is crystallized from methanol. 1.2 g of N - cyano - N' - [2 - [[[2 - [(aminoiminomethyl)amino] - 4-thiazolyl]methyl]thio]ethyl]formamidine acid maleate.

Melting point: 178—179°C.

One basic group (anhydrous medium): 99.9%

| Elemental analysis: | C | H | N |
|---|---|---|---|
| found | 39.12 | 4.61 | 23.89 |
| calculated | 39.09 | 4.29 | 24.55 |

IR Spectrum: characteristic bands at 2190 $cm^{-1}$ (C≡N st) and 1615 $cm^{-1}$ (C=N st).

Example 4

N-Cyano-N'-[3-[3-(1-piperidinylmethyl)phenoxy]propyl]formamidine hydrochloride

I. R = 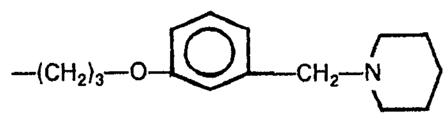 · HCl

To a solution of 2.48 g of 3 - [3 - (1 - piperidinylmethyl)phenoxy] - 1 - propanamine (prepared according to British Patent No. 2,023,123) in 25 ml of absolute ethanol, 0.98 g of ethyl N-cyano-formimidate in 15 ml of ethanol are added dropwise with stirring and at room temperature. Stirring is continued for 30 minutes and the ethanol is removed by distillation under reduced pressure. An oily residue is formed which is dissolved in 250 ml of ethyl ether and 0.95 ml of 10N HCl/ethanol are added, thus yielding 1.4 g of N - cyano - N' - [3 - [ 3 - (1 - piperidinylmethyl)phenoxy]propyl]formamidine hydrochloride.

Melting point: 57—61°C.

One basic group (anhydrous medium): 101.6%

| Elemental analysis: | C | H | N |
|---|---|---|---|
| found | 59.03 | 7.78 | 14.89 |
| calculated | 60.61 | 7.48 | 16.63 |

| Chlorides: | found | calculated |
|---|---|---|
| | 10.52 | 10.84 |

IR Spectrum: characteristic bands at 2190 $cm^{-1}$ (–C≡N st) and 1620 $cm^{-1}$ (—C=N st).

**Claims**

1. N-Cyano-formamidine compounds of the general formula I:

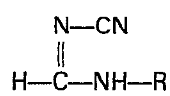 I

wherein R is a radical of the general formulae:

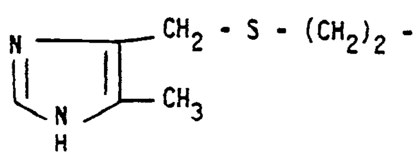

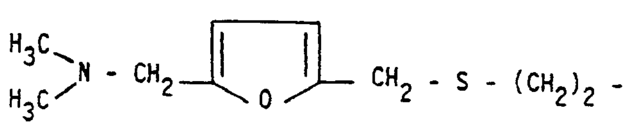

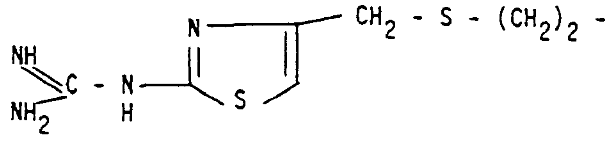

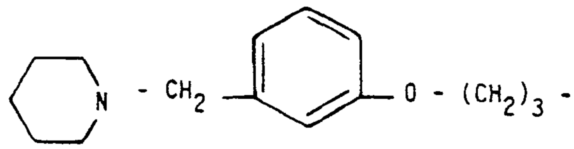

2. Process for preparing the compounds according to claim 1 characterized in that a compound of the general formula II

$$\begin{array}{c} N{-}CN \\ \| \\ H{-}C{-}OR_1 \end{array} \qquad \text{II}$$

wherein $R_1$ is a linear or branched $C_1$—$C_4$ alkyl group, is reacted with an amine of the general formula III

$$R{-}NH_2 \qquad \text{III}$$

wherein R is as defined in claim 1, in a reaction inert organic medium and the so obtained compound optionally is converted into a non-toxic acid addition salt thereof.

3. The process according to claim 2, characterized in that acetonitrile or a $C_1$—$C_4$ alkanol is used as reaction medium.

4. The process according to claims 2 or 3, characterized in that ethanol is used as reaction medium.

5. Pharmaceutical composition comprising at least one compound according to claim 1, optionally in combination with pharmaceutically acceptable carriers and/or adjuvants.

**Patentansprüche**

1. N-Cyano-formamidin-Verbindungen der allgemeinen Formel I:

$$\begin{array}{c} N{-}CN \\ \| \\ H{-}C{-}NH{-}R \end{array} \qquad \text{I}$$

worin R einen Rest der allgemeinen Formeln:

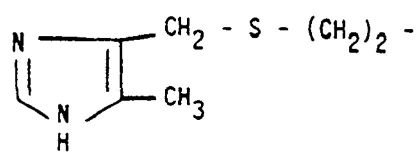

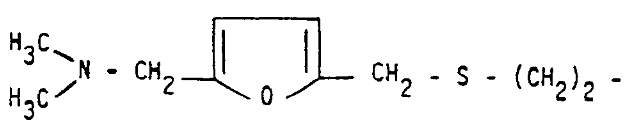

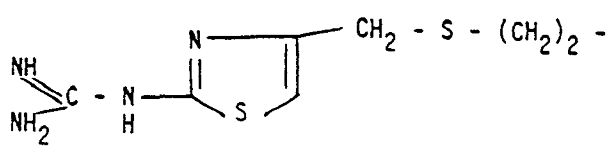

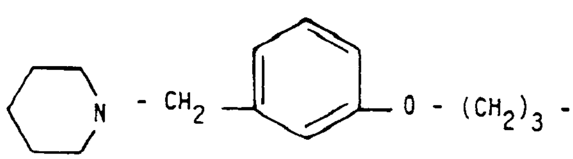

bedeutet,
und deren nicht-toxische Säureadditionssalze.

2. Verfahren zur Herstellung der Verbindungen nach Anspruch 1, dadurch gekennzeichnet, daß man eine Verbindung der allgemeinen Formel II:

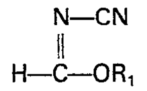

II

worin $R_1$ eine geradkettige oder verzweigte $C_1$—$C_4$-Alkylgruppe bedeutet, mit einem Amin der allgemeinen Formel III

$$R—NH_2 \qquad III$$

worin R die in Anspruch 1 angegebenen Bedeutungen besitzt, in einem reaktionsinerten organischen Medium umsetzt und die so erhaltene Verbindung gegebenenfalls in ein nichttoxisches Säure-Additionssalz überführt.

3. Verfahren nach Anspruch 2, dadurch gekennzeichnet, daß man als Reaktionsmedium Acetonitril oder ein $C_1$—$C_4$-Alkanol verwendet.

4. Verfahren nach Anspruch 2 oder 3, dadurch gekennzeichnet, daß man als Reaktionsmedium Äthanol verwendet.

5. Pharmazeutisches Mittel, enthaltend wenigstens eine Verbindung nach Anspruch 1, gegebenenfalls in Kombination mit pharmazeutisch verträglichen Trägern und/oder Adjuvantien.

**Revendications**

1. Composés nitro-cyanoformamidins de la formule générale I:

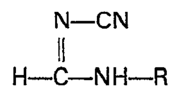

I

R étant un radical des formules générales:

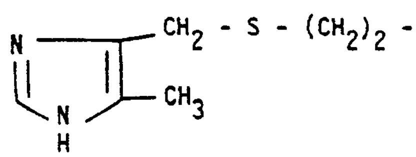

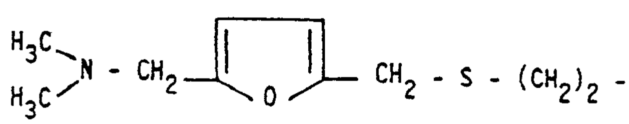

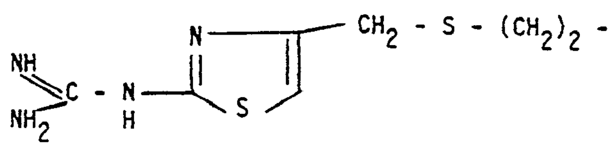

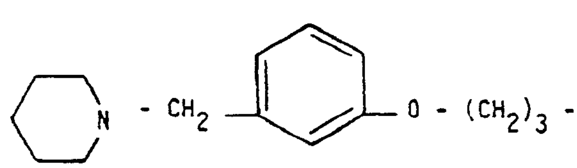

2. Procédé de préparation des composé selon revendication 1, caractérisé en ce qu'un composé de la formule générale II

$$\begin{array}{c} N{-}CN \\ \| \\ H{-}C{-}OR_1 \end{array} \qquad II$$

dans laquelle $R_1$ est un groupe alkyle $C_1$—$C_4$ linéaire ou ramifié, est mis en réaction avec une amine de la formule générale III

$$R{-}NH_2 \qquad III$$

R étant tel que défini à la revendication 1, dans un milieu organique neutre et le composé ainsi obtenu étant facultativement converti en un sel d'addition acide non-toxique de celle-ci.

3. Procédé selon revendication 2, caractérisé en ce que l'acétonitrile ou un alkanol $C_1$—$C_4$ est utilisé comme milieu réactionnnel.

4. Procédé selon revendication 2 ou 3, caractérisé en ce que l'éthanol est utilisé comme milieu réactionnel.

5. Composition pharmaceutique comprenant au minimum un composé selon revendication 1, combiné facultativement avec des véhicules et/ou adjuvants acceptables en pharmacie.